(19) Europäisches Patentamt
European Patent Office
Office européen des brevets

(11) Veröffentlichungsnummer : **0 379 981 B1**

(12) # EUROPÄISCHE PATENTSCHRIFT

(45) Veröffentlichungstag der Patentschrift : **07.04.93 Patentblatt 93/14**

(51) Int. Cl.$^5$ : **C07C 43/307,** C07D 317/12, A61K 7/46

(21) Anmeldenummer : **90101047.0**

(22) Anmeldetag : **19.01.90**

(54) **Acetale von Oxo-tetralinen und von Oxo-indanen.**

(30) Priorität : **27.01.89 CH 258/89**
**30.11.89 CH 4305/89**

(43) Veröffentlichungstag der Anmeldung : **01.08.90 Patentblatt 90/31**

(45) Bekanntmachung des Hinweises auf die Patenterteilung : **07.04.93 Patentblatt 93/14**

(84) Benannte Vertragsstaaten : **CH DE ES FR GB IT LI NL**

(56) Entgegenhaltungen :
**EP-A- 0 040 338**
**EP-A- 0 301 375**
**EP-B- 0 035 305**
**CH-A- 618 344**
**DE-B- 2 427 465**
**DE-C- 3 502 188**

(73) Patentinhaber : **L. GIVAUDAN & CIE Société Anonyme**
**CH-1214 Vernier-Genève (CH)**

(72) Erfinder : **Bonenfant, Alain-Pierre**
**2 Rue Edouard Rod**
**CH-1203 Genf (CH)**
Erfinder : **Baudin, Josianne**
**6 Rue René Naudin**
**F-74100 Annemasse (FR)**
Erfinder : **Gonzenbach, Hans Ulrich**
**61bis Rue de Lyon**
**CH-1203 Genf (CH)**

(74) Vertreter : **Urech, Peter, Dr. et al**
**Grenzacherstrasse 124 Postfach 3255**
**CH-4002 Basel (CH)**

EP 0 379 981 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft neue Riechstoffe, nämlich Acetale, bzw. nach der alten Nomenklatur Acetale und Ketale von alkylsubstituierten Oxo-tetralinen und Oxoindanen, beispielsweise von Oxo-tetralinen oder Oxo-indanen mit bis zu 6 oder 7 Alkylsubstituenten.

Die Erfindung betrifft, genauer gesagt, die Verbindungen der Formel

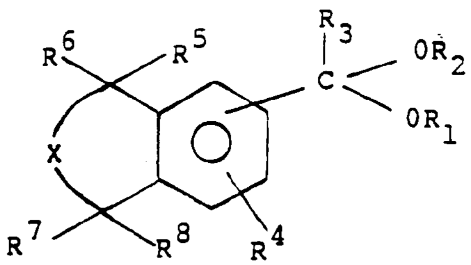

I

worin bedeuten:

| | |
|---|---|
| $R^1$, $R^2$ | $C_{1-4}$-Alkyl, $C_{2-4}$-Alkenyl |
| | oder |
| $R^1 + R^2$ | $C_{2-4}$-Alkylen, gegebenenfalls $C_{1-4}$-alkyl-, $C_{2-4}$-alkenyl-oder hydroxy-$C_{1-4}$-alkyl- bzw. hydroxy-$C_{2-4}$-alkenyl-substituiert |
| $R^3$ | H, $CH_3$ |
| $R^4$ | H, $C_{1-4}$-Alkyl |
| $R^5$, $R^6$, $R^7$, $R^8$, | H, $CH_3$, $C_2H_5$, $CH_2CH_2CH_3$, $CH(CH_3)_2$ |
| X | Methylen, Aethyliden, Propyliden, Aethylen, Propylen, 2,3-Butyliden |

Gesamtzahl der Kohlenstoffatome von $R^1$, $R^2$ $\leqslant 6$

Gesamtzahl der Kohlenstoffatome von $R^1$, $R^2$, $R^3$, $R^4$ $\leqslant 11$

Gesamtzahl der Kohlenstoffatome von $R^5$, $R^6$, $R^7$, $R^8$ $\leqslant 6$

mit der Ausnahme derjenigen Verbindungen I, worin

| | |
|---|---|
| $R^3$ | H |
| $R^4$ | H oder $CH_3$ |
| $R^5$, $R^6$, $R^7$, $R^8$ | $CH_3$ |
| X | Aethylen |
| | und |
| $R^1$, $R^2$ | $C_{1-4}$-Alkyl |

Als Untergruppen fungieren dementsprechend:

1. Die offenkettigen Acetale und Ketale der Oxo-tetraline der Formel I.
2. Die offenkettigen Acetale und Ketale der Oxo-indane der Formel I.
3. Die cyclischen Acetale und Ketale der Oxo-indane der Formel I.
4. Die cyclischen Acetale und Ketale der Oxo-tetraline der Formel I.

Die Verbindungen der Formel I zeichnen sich durch sehr natürliche Noten in Richtung Moschus, Amber, holzig, animalisch und sogar fruchtig aus.

Im allgemeinen herrschen in den "leichteren" Molekülen Fruchtnoten vor. Die schwereren, z.B. cyclischen Moleküle verfügen über Holz-, Moschus- und animalische Noten.

Detailliert gilt folgendes:

| | | |
|---|---|---|
| Acetale | einfachere, offenkettige | Moschus, holzig, fruchtig, camphrig, |
| | cyclische | Moschus betonter, ohne Camphernote |
| | substituierte | s.u. |
| Ketale | einfachere, offenkettige | Moschus, holzig, stärker fruchtig, weniger camphrig als entsprechende Acetale |
| | cyclische | stark holzig |
| | substituierte | komplexere Aspekte, geruchsstark: holzig/Moschus/fruchtig/Ambra/animalisch |

Aus ökonomischen Gründen sind die Tetraline bevorzugt.

Verglichen mit den zugrunde liegenden Carbonylderivaten, die ebenfalls Riechstoffe sind, siehe z.B. EP-A 301375, 040338, DE-B-2427465, sind sie im allgemeinen stärker, holziger, animalischer, substantiver.

Ein beträchtlicher technischer Vorteil in der Verwendung von I ergibt sich daraus, dass sie beim Mischen mit dem "Grundkörper" oft flüssige Gemische ergeben. Man kann also mittels der neuen Verbindungen I bekannte Moschuskörper, insbesondere jeweils den entsprechenden Grundkörper, durch genaue Dosierung auf diese Weise quasi veredeln, verfeinern, ästhetisieren, Moschuskörper variabler Intensität bzw. verschiedener geruchlicher Nuancierung herstellen. Solche Gemische, also Aldehyde oder Ketone II der in der Folge angegebenen Formeln und entsprechendes Acetal oder Ketal I eignen sich dank ausgesprochener Substantivität und Haftfestigkeit insbesondere auch zur Parfümierung von Wäsche bzw. von Textilveredlern, siehe diesbezüglich die untenstehende Tabelle III.

Das optimale Verhältnis von Verbindung I/einem "Grundkörper" ergibt sich jeweils aufgrund der Ergebnisse von Waschversuchen und natürlich auch Löslichkeit des "Grundkörpers" in der Verbindung I. Es liegt beispielsweise zwischen ca. 1:10 bis 1:1, aber auch extremere Werte sind möglich.

Die Herstellung der Kompositionen geschieht auf die übliche Weise, also durch Mischen, wie unten angegeben.

Bevorzugte Verbindungen der Formel I sind insbesondere:
2,4-Dimethyl-2-[1,1,4,4-tetramethyl-tetralin-6-yl]-1,3-dioxolan,
2-Methyl-4-äthyl-2-[1,1,4,4-tetramethyl-tetralin-6-yl]-1,3-dioxolan,
7-[Formyl-diethyl-acetal]-1,1,2,4,4-pentamethyltetralin,
2,4-Dimethyl-2-[1,1,4,4-tetramethyl-tetralin-6-yl]-1,3-dioxan,
6-(Acetyl-diethyl-ketal)-1,1,2,4,4,7-hexamethyl-tetralin,
2,4-Dimethyl-2-[1,1,2,4,4,7-hexamethyl-tetralin-6-yl]-1,3-dioxolan.

Die im Vordergrund des Interesses stehenden Verbindungen der Formel I werden dadurch erhalten, dass man eine Verbindung der Formel

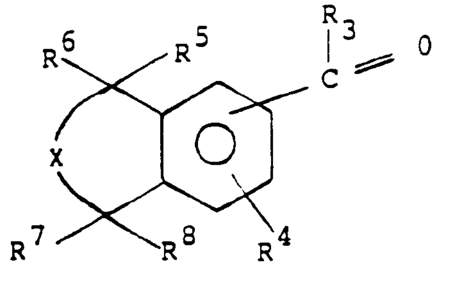

II

in an sich bekannter Weise acetalisiert bzw. ketalisiert,
oder eine Verbindung I transacetalisiert bzuw. ketalisiert,
oder eine Verbindung der Formel

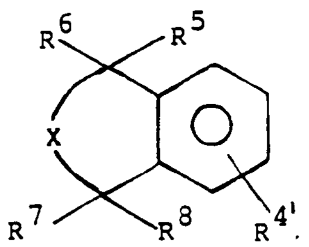

III

worin $R^{4'}$ Methyl oder Aethyl bedeutet,
in Gegenwart eines Alkohols $R^{2'}OH$, und zwar eines $C_{1-3}$-Alkanols und eines Leitsalzes elektrochemisch oxidiert.

Die Methodik zur Herstellung von Acetalen und Ketalen ist bekannt, siehe z.B.
A.J. Meskens, Synthesis (1981), 501 seq.
oder auch DT-OS 2848397.

Die Herstellung erfolgt also beispielsweise nach den an sich bekannten Methoden:
A Verbindung II + Orthoameisensäureester (die schonendste Methode)
B Verbindung II + Alkohol (die universellste Methode)
C Verbindung II + Diol (für die Cyclen)
D Transacetalisierung, Transketalisierung einer Verbindung I
E elektrochemisch.

Für diese Methoden gilt insbesondere noch:
A.

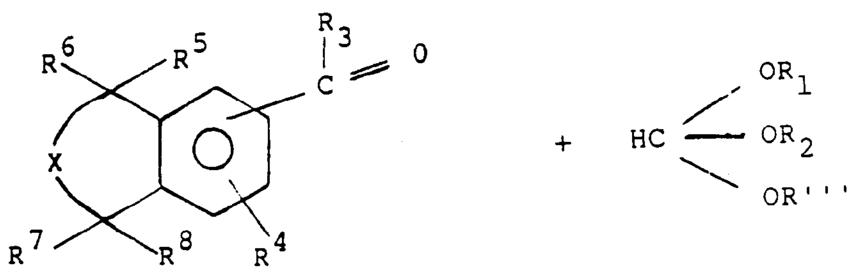

II

$R^1$, $R^2$ $C_{1-4}$-Alkyl

R''' entspricht zweckmässigerweise $R^1$, $R^2$

<u>Parameter</u>

| | |
|---|---|
| Temperaturbereich: | ca. 10°C bis ca. 110°C, insbesondere ca. 20°C - ca. 40°C |
| zusätzliche Lösungsmittel: (fakultativ) | Alkanole, Hexan, Cyclohexan, etc. |

| | |
|---|---|
| Katalysatoren: | Säuren, z.B. HCl, p-Toluolsulfonsäure, $Al_2O_3 \cdot 4SiO_2$ .n $H_2O$), saure Salze, z.B. $KHSO_4$, saure Ionentauscher |
| Isolation der Verbindung: | Destillation unter vermindertem Druck, Kolonnenchromatographie |

B.

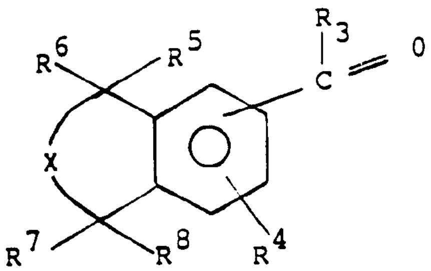

+ $C_{1-4}$-Alkanol, $C_{2-4}$-Alkenol

II

<u>Parameter</u>

| | |
|---|---|
| Temperaturbereich: | ca. 10°C - ca. 100°C |
| Zusätzliche Lösungsmittel: | $CH_2Cl_2$, $C_6H_6$, Toluol, etc. |
| Katalysatoren: | (Lewis-)Säuren, z.B. HCl, $H_2SO_4$, $HNO_3$, Toluolsulfonsäure, $BF_3$, Sulfosalicylsäure, saure Salze, z.B. $KHSO_4$, saure Ionentauscher |
| Isolation der Verbindung I: | Destillation unter vermindertem Druck, Kolonnenchromatographie |

C.

Umsatz von II mit einem entsprechenden, gegebenenfalls substituierten Diol, z.B. Aethan-diol, 1,2-Propan-diol, 1,3-Propan-diol, 1,2-Butan-diol, 2,3-Butan-diol, 1,3-Butan-diol, 2-Buten-1,4-diol, 2-Methyl-1,4-butan-diol, etc.

Die Methode ist natürlich auch auf Polyole, z.B. Glycerin, 1,2,3-Pentan-triol, 1,2,3,4-Butan-tetraol, 1,2,4-Butan-triol und Isomere davon, etc. anwendbar.

<u>Parameter</u>

| | |
|---|---|
| zusätzliche Lösungsmittel: (fakultativ) | Aether, (chlorierte) Kohlenwasserstoffe |
| Katalysatoren: | Säuren, inkl. Lewissäuren, saure Salze, etc. |
| Temperaturbereich: | ca. 30°C bis ca. 150°C. |
| Isolierung: | Destillation, Kristallisation |

D.

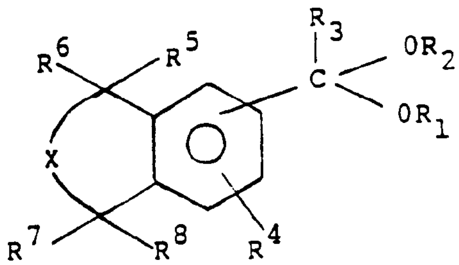

+ Alkohol Höh.

I

Mit der Transacetalisierung bzw. Transketalisierung von I wird insbesondere der Rest $R^1$ bzw. $R^2$ eines niedriger siedenden Alkohols durch den Rest eines höher siedenden Alkohols Höh. ersetzt, wobei H auch ein Diol sein kann, und wobei das Gleichgewicht bekanntlich auch von der Menge des eingesetzten Alkohols abhängt.

<u>Parameter</u>

| | |
|---|---|
| Temperaturbereich: | ca. 65°C bis 85°C |
| Zusätzliche Lösungsmittel: | Toluol, etc. |
| Isolation der Verbindung I: | z.B. durch Destillation |

E.

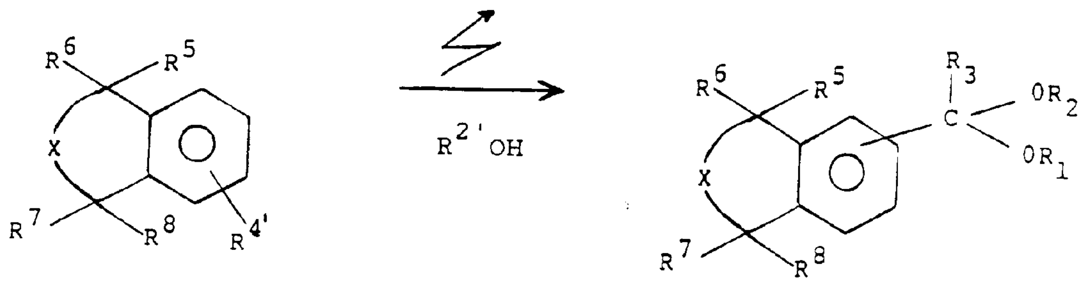


III

I

$R^{4'}$ = $CH_3$, $C_2H_5$

$R^{2'}OH$ = $C_{1-3}$-Alkanol, $C_{1-3}$-Alkandiol

In der Verbindung I entsprechen die Reste $R^1$ und $R^2$ dem eingesetzten Alkohol $R^{2'}OH$.

Im Falle der elektrochemischen Methode wird zweckmässigerweise vorgegangen wie z.B. in der DT-OS 2848397 detailliert beschrieben.

Zweckmässige Parameter sind demzufolge zum Beispiel:

| | |
|---|---|
| Zelle | : geteilt oder ungeteilt |
| Elektrolyt | : Lösung von III im verwendeten Alkohol, die ein Leitsalz enthält |
| Leitsalz | : die in der Elektrochemie üblichen Leitsalze. Gut geeignet sind z.B. Salze, die in der zu elektrolysierenden Lösung löslich und unter den Versuchsbedingungen weitgehend stabil sind. Beispiele besonders geeigneter Leitsalze sind Fluoride, wie KF, Tetrafluoroorate, wie $Et_4NBF_4$, Perchlorate, wie $Et_4NClO_4$, Sulfate, wie $Et_4NSO_4Et$, Alkoholate, wie $NaOCH_3$ und Hydroxide, wie KOH. |
| Anodenmaterial | : Beispiele für geeignete Anodenmaterialien sind Graphit, graphitgefüllte Kunststoffe, Edelmetalle, wie Platin und Gold sowie edelmetallbeschichtete Titanelektroden. |
| Kathodenmaterial | : beispielsweise Graphit-, Eisen-, Stahl-, Blei- oder Edelmetallelektroden. |
| Stromdichte: | : beispielsweise 1-5 $A/dm^2$. |

Temperatur : z.B. Temperaturen zwischen O und ca. 60°C.
Aufarbeitung : Die Elektrolyseausträge werden in der Regel destillativ aufgearbeitet. Ueberschüssiger Alkohol und evtl. noch vorhandenes Ausgangsmaterial werden von den Acetalen durch Destillation abgetrennt und können zur Elektrolyse zurückgeführt werden.

Beispiele

Allgemeine Methodik

Methode A

0,02 Mol Acetylchlorid werden tropfenweise innert ungefähr 5 Minuten zu einem Gemisch des Orthoformiatesters (Aethyl- oder Methylester: 0.763 Mol) 0,6 Mol Aethanol oder Methanol und 0,28 Mol Aldehyd oder Keton gegeben. Die Reaktion ist leicht exotherm, wobei die Temperatur von ca. 20°C auf ca. 40°C ansteigt. Das resultierende Gemisch wird 2 Std. bei Raumtemperatur gerührt, hierauf wird bis zu einem pH von 7.5 bis 8 alkalisch gemacht, und zwar durch Zugabe einer 10%igen Lösung von Kaliumhydroxid in Aethanol. Nach Filtrieren, Entfernen des Orthoformiats und des Alkohols unter vermindertem Druck wird durch Destillation das reine Acetal, beispielsweise das Diäthyl- oder Methylacetal erhalten.

Methode B

1,5 mMole Acetylchlorid werden innert 5 Min. tropfenweise zu einem Gemisch von 0,075 Mol Propanol und 0.022 Mol Aldehyd gegeben. Das Reaktionsgemisch wird auf 65°C erhitzt und 3 Std. bei dieser Temperatur gerührt. Nach Abkühlen auf Raumtemperatur wird das Reaktionsgemisch durch Zugabe einer 10%igen Lösung von Kaliumhydroxyd in Aethanol neutralisiert. Das Reaktionsgemisch wird filtriert, und es wird abgedampft, wobei das rohe Acetal anfällt. Dieses wird durch Chromatographie an Kieselgel gereinigt, wobei von Isopropyläther/Hexan im Verhältnis von 1:20 als Eluen Gebrauch gemacht wird.

Methode C

0,1 Mol Aldehyd oder Keton, 0,2 Mol Diol (beispielsweise Aethylenglycol oder Propylenglycol), 200 ml Toluol und 0,15 g p-Tuolsulfonsäuremonohydrat werden gerührt und erhitzt. Das während der Reaktion gebildete Wasser wird innerhalb 24 Std. azeotrop abdestilliert. Durch Zugabe einer 10%igen Lösung von Kaliumhydroxyd in Aethanol wird das Reaktionsgemisch auf einen pH-Wert von 7,5 bis 8 gebracht. Hierauf wird filtriert. Nach Abdampfen des Lösungsmittels wird das rohe Acetal durch Destillation oder Rekristallisation gereinigt.

2,4-Dimethyl-2-[1,1,2,4,4,7-hexamethyl-tetralin-6-yl]-1,3-dioxolan

Dieselbe Methode gestattet, dieses Dioxolan ausgehend aus 6-Acetyl-1,1,2,4,4,7-hexamethyl-tetralin und Propylenglykol herzustellen; Sdp. 109°C/0,2 mm Hg, $nD^{20}$ = 1,5182. Geruch: moschusartig, grün, weich, pudrig.

Methode D

a) 0,1 g p-Toluolsulfonsäure werden zu einem Gemisch von 0.066 Mol Formylacetal, z.B. 6-Formyl-1,1,3,4,4-pentamethyl-tetralinediäthylacetal gegeben und auch noch 0,066 Mol Allylalkohol und 50 ml Toluol zugegeben. Das resultierende Gemisch wird 4 Std. bei 85°C bis 90°C gerührt. Das bei der Reaktion gebildete Aethanol wird durch Destillation entfernt. Hierauf wird durch Zugabe von 10%igem Kaliumhydroxyd in Aethanol neutralisiert, filtriert und abgedampft, wobei das gebildete Acetal, beispielsweise Allylaethylacetat, erhalten wird. Die Isolation geschieht durch präparative Gaschromatrographie.
b) 0,1 g Acetylchlorid werden tropfenweise innert 5 Min. zu einem Gemisch von 0,35 Mol Aethylformiat, 0,31 Mol Aethanol und 0,13 Mol Keton gegeben. Die Reaktion ist leicht exotherm, was sich in einem Temperaturanstieg von 20°C auf 28°C auswirkt. Das resultierende Gemisch wird 2 Std. bei Zimmertemperatur gerührt. Der Ueberschuss an Orthoformiat wird durch Destillation entfernt und zwar im Vakuum bei beispielsweise 50 mm Hg. Das Reaktionsgemisch wird gekühlt und 50 ml Toluol und 0,20 Mol Propylenglycol zugegeben, ebenso noch 1,5 mMol p-Toluolsulfonsäure. Das resultierende Gemisch wird 2 1/2 Std. bei 85°C bis 90°C gerührt. Durch Destillation wird das gebildete Ethanol entfernt. Das Gemisch wird neutralisiert, und zwar durch Zugabe von 10% Kaliumhydroxyd in Aethanol; es wird filtriert und abgedampft, wobei das rohe Acetal anfällt, welches durch Destillation gereinigt werden kann.

Beispiel 1.A

7-[Formyl-diäthylacetal]-1,1,2,4,4-pentamethyl-tetralin

0,02 Mol (= 1,65 g) Acetylchlorid werden tropfenweise innert 1 Min. zu einem gerührten Gemisch von 113 g (0,76 Mol) Aethylorthoformiat, 35 ml Aethanol und 64.3 g (0,28 Mol) 7-Formyl-1,1,2,4,4-Pentamethyl- tetralin bei Zimmertemperatur gegeben. Die Reaktion ist leicht exotherm, was sich durch einen Temperaturanstieg von 20°C auf 38°C auswirkt. Das resultierende Gemisch wird 2 Std. bei Raumtemperatur gerührt, nach welcher Zeit durch Zugabe einer 10%igen Lösung von Kaliumhydroxyd in Ethanol auf ein pH von 7,5 bis 8 gestellt wird. Nach Filtrieren und Entfernen des überschüssigen Aethanols und des Aethylorthoformiats durch Destillation wird die zurückbleibende Flüssigkeit vakuumdestilliert, wobei zur Hauptsache 69 g 7-[Formyl-diethylacetal]-1,1,2,4,4-pentamethyl-tetralin anfallen und zwar als farblose Lösung. Siedepunkt 112°C/0.25 mmHg; $nD^{20}$ 1,5015. Der Geruch ist leicht moschusartig.

Beispiel 1.B

7-[Formyl-dipropylacetal]-1,1,2,4,4-pentamethyl-tetralin

Zu einem Gemisch von 10 g (0,04 Mol) 7-Formyl-1,1,2,4,4-pentamethyl-tetralin und 10 g (0,17 Mol) n-Propanol werden bei Zimmertemperatur tropfenweise 0,25 g Acetylchlorid zugegeben. Das Reaktionsgemisch wird auf 65°C erhitzt und 2 Std. bei dieser Temperatur gerührt. Nach Abkühlen auf Raumtemperatur wird das Reaktionsgemisch neutralisiert, und zwar durch Zugabe von 10%igen Kaliumhydroxyd in Ethanol. Das Gemisch wird filtriert und es wird abgedampft, wobei das rohe 7-[Formyl-dipropylacetal]-1,1,2,4,4-pentamethyl-tetralin anfällt, welches Produkt durch Chromatographie an Silicagel gereinigt wird. Man macht dabei von Isopropyläther/Hexan im Verhältnis von 1:20 als Eluens Gebrauch. Das Acetal stellt eine farblose Flüssigkeit dar; $nD^{20}$ = 1,4980. Der Geruch ist ausgeprägt moschusartig.

Beispiel 1.C

2,4-Dimethyl-2-[1,1,4,4-tetramethyl-tetralin-6-yl]-1,3-dioxolan

Zu einer Suspension von 120 g (0,5 Mol) 6-Acetyl-1,1,4,4-tetramethyl-tetralin, 120 g (1,6 Mol) Propylenglycol und 600 ml Toluol werden 2 g p-Toluolsulfonsäuremonohydrat gegeben. Man erhitzt das Gemisch auf 110°C. Innert 5 Std. wird das Reaktionswasser azeotrop entfernt. Nach Kühlen auf Zimmertemperatur wird durch Zugabe von 10% Kaliumhydroxyd in Aethanol auf pH 7,5 bis 8 gestellt. Hierauf wird filtriert. Man dampft das Lösungsmittel ab und destilliert die zurückbleibende Flüssigkeit unter Vakuum, wobei als Hauptprodukt 123 g 2,4-Dimethyl-2[1,1,4,4-tetramethyl-tetralin-6-yl]-1,3-dioxolan anfallen und zwar als Gemisch der cis-trans-Isomeren. Der Siedepunkt ist 112°C bei 0,5 mmHg; $nD^{20}$ = 1.5130. Das Produkt hat einen stark holzigen, animalischen Geruch.

Beispiel 1.D

2,4-Dimethyl-2-[1,1,4,4-tetramethyl-tetralin-6-yl]-1,3-dioxolan

0,15 g Acetylchlorid werden bei Zimmertemperatur tropfenweise zu einem Gemisch von 52,5 g (0,35 Mol) Aethylorthoformiat, 30 g (0,13 Mol) 6-Acetyl-1,1,4,4-tetramethyl-tetralin und 18 ml Aethanol gegeben. Die Reaktion ist leicht exotherm, was sich in einem Anstieg der Temperatur von 20°C auf 28°C auswirkt. Man rührt 2 Std. bei dieser Temperatur. Der Ueberschuss an Aethylorthoformiat und Ethanol wird durch Vakuumdestillation (120°C/100 mm Hg) entfernt. Nach dieser Zeit wird das Reaktionsgemisch gekühlt und 50 ml Toluol, 22,5 g (0,29 Mol) Propylenglycol, 0,3 g p-Toluolsulfonsäure werden schnell zugegeben. Das Gemisch wird 2 1/2 Std. bei 85°C bis 90°C gerührt. Das gebildete Aethanol wird destillativ entfernt. Hierauf wird das zurückbleibende Gemisch durch Zugabe einer 10%igen Lösung von Kaliumhydroxyd in Ethanol neutralisiert, filtriert und es wird eingedampft. Es entsteht das rohe 2-Dimethyl-2-[1,1,4,4-tetramethyl-tetralin-6-yl]-1,3-dioxolan. Das Produkt wird durch Vakuumdestillation gereinigt, wobei 23,7 g reines Produkt anfallen. Der Siedepunkt des reinen Produktes ist 112° bei 0,45 mmHg.

Beispiel 1.E

In einem 3 L Vierhalsgefäss mit Heizung, Bodenhahn und Rückflusskühler werden 200 g 1,1,2,4,4,7-Hexamethyl-tetralin, gelöst in 500 g Cyclohexan (wasserfrei) vorgelegt und 1 kg Ethanol (wasserfrei), enthaltend

14 g Schwefelsäure (98%) unter Rühren zugegeben. Dieses Gemisch wird mittels einer Pumpe (10 l/min) durch die Elektrolysezelle (Typ Filterpresse mit Graphitanoden, Inoxkathoden im Abstand von 6 mm, wirksame Oberfläche: 220 $cm^2$) umgewälzt (Potential-differenz 35 - 42 Volt bei 3,5 - 4 Ampère, Dauer 38 Stunden, Reservoirtemp. 25 - 30°C). Eine GC-Analyse ergibt einen Gehalt der gewünschten Verbindung, nämlich 7-[Formyl-diäthylacetal]-1,1,2,4,4-pentamethyl-tetralin von 77%. Nach Zugabe von 18,5 g Natriumkarbonat wird eingedampft (40°C, 20 mmHg). Die verbleibenden 285 g werden mit 300 g Kochsalzlösung (10%) gewaschen und über eine Vigreuxkolonne destilliert (130°C, 3mmHg). Ausbeute: 224 g, 87%, Reinheit 83% (GC) Hauptverunreinigung: 1,1,2,4,4-Pentamethyl-7-formyl-tetralin (GC 11%).

Es werden weitere Verbindungen gemäss Tabelle I hergestellt.

Tabelle I

a) Ketale (offenkettig, $R^3 = CH_3$)

| Ausgangsmaterial | $R^1$ | $R^2$ | Methode | Sdp. °C (mmHg) | Smp. °C | $n_D^{20}$ | Geruch |
|---|---|---|---|---|---|---|---|
| 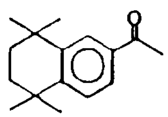 | $CH_3$ | $CH_3$ | A | | 44.5-46 | | holzig, Amber |
| | $C_2H_5$ | $C_2H_5$ | A | $93_{(0.1)}$ | | 1.4985 | leicht holzig |
| 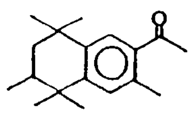 | $CH_3$ | $CH_3$ | A | | 80-81.5 | | Moschus, Amber, animalisch |
| | $C_2H_5$ | $C_2H_5$ | A | | 85-87 | | Moschus |
| 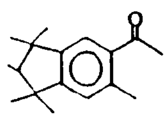 | $C_2H_5$ | $C_2H_5$ | A | | 62-63 | | Moschus, holzig |
| 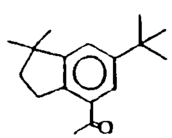 | $C_2H_5$ | $C_2H_5$ | A | $94_{(0.2)}$ | | 1.4945 | Moschus, animalisch |

b) Acetale (offenkettig, $R^3$ = H)

| | | | | | | | |
|---|---|---|---|---|---|---|---|
| 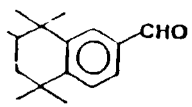 | $CH_3$ | $CH_3$ | A | $103_{(0.1)}$ | | 1.5120 | leicht nach Moschus, blumig |
| | $C_2H_5$ | $C_2H_5$ | A | $112_{(0.25)}$ | | 1.5015 | leicht nach Moschus, blumig |
| | $C_3H_7$ | $C_3H_7$ | B | | | | nach Moschus |
| | Allyl | Allyl | D (a) | | | | nach Moschus |
| 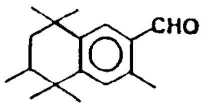 | $CH_3$ | $CH_3$ | A | | 50-51 | | nach Moschus |
| | $C_2H_5$ | $C_2H_5$ | A | $108_{(0.1)}$ | | 1.5030 | nach Moschus, pudrig |
| 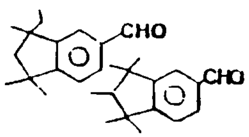 | $CH_3$ | $CH_3$ | A | $146_{(9.5)}$ | | 1.5045 | nach Moschus, weich, blumig |
| | $C_2H_5$ | $C_2H_5$ | A | $98_{(0.1)}$ | | 1.4950 | leicht nach Moschus |
| 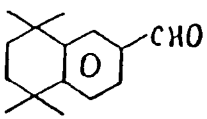 | $C_2H_5$ | $C_2H_5$ | A | $145_{(0.05)}$ | | | nach Honig, grün, blumig |

Tabelle II

a) Ketale 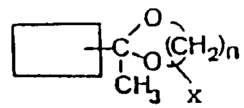 $C_{x+n} \nless 6$

| Ausgangs-material | | n | x | Methode | Sdp. °C (mmHg) | Smp. °C | $n_D^{20}$ | Geruch |
|---|---|---|---|---|---|---|---|---|
| 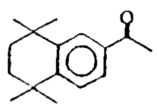 | $CH_2OHCH_2OH$ | 2 | H | C | | 77 | | holzig, nach Moschus |
| | $CH_2OHCHOHCH_2OH$ | 2 | $CH_2OH$ | C | $180_{(0.04)}$ | | 1.5265 | holzig, animalisch |
| | $CH_2OHCHOHCH_3$ | 2 | $CH_3$ | C, E | $112_{(0.45)}$ | | 1.5130 | holzig, animalisch |
| 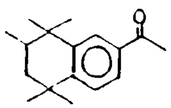 | $CH_2OHCH_2OH$ | 2 | H | C | | 111-112 | | nach Moschus, animalisch |
| 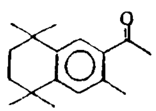 | $CH_2OHCH_2OH$ | 2 | H | C | | 112 | | leicht nach Moschus |

| | | | | | | | | |
|---|---|---|---|---|---|---|---|---|
| 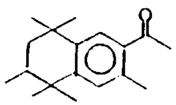 | $CH_2OHCH_2OH$ | 2 | H | C | | 119-120 | | nach Moschus, Tonka |
| | $CH_3CHOHCH_2OH$ | 2 | $CH_3$ | C | $109_{(0.2)}$ | | 1.5182 | nach Moschus, grün, pudrig, süss |
| 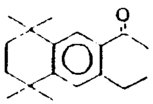 | $CH_2OHCH_2OH$ | 2 | H | C | | 110-111 | | leicht nach Moschus |
| 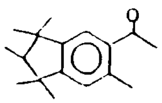 | $CH_2OHCH_2OH$ | 2 | H | C | | 108-110 | | nach Moschus, animalisch |
| 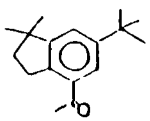 | $CH_2OHCH_2OH$ | 2 | H | C | | 71-72 | | holzig, nach Moschus |
| | $CH_3CHOHCH_2OH$ | 2 | $CH_3$ | C | $109_{(0.5)}$ | | 1.5065 | holzig, animalisch |
| 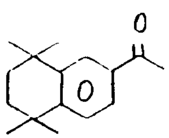 | $CH_2OHCHOHCH_2CH_3$ | 2 | $C_2H_5$ | C | $109_{(0.5)}$ | | | holzig, Amber, animalisch, Tabak |
| | $CH_3CHOHCHOHCH_3$ | 2 | $CH_3$, $CH_3$ | C | $111_{(0.3)}$ | | | holzig, Amber, nach Moschus |
| | $CH_2OH-CH_2-CH_2OH$ | 3 | H | C | $94_{(0.15)}$ | | | leicht holzig, leicht fettig |
| | $CH_2OH-CH_2-CHOH-CH_3$ | 3 | $CH_3$ | C | | 90-92 | | nach Moschus, Amber, holzig, animalisch |

| | | | | | | | |
|---|---|---|---|---|---|---|---|
| 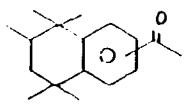 | $CH_2OHCHOHCH_3$ | 2 | $CH_3$ | C | $100_{(0.08)}$ | | holzig, animalisch |
| 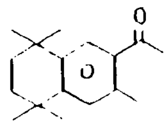 | $CH_2OHCHOHCH_3$ | 2 | $CH_3$ | C | $122_{(0.9)}$ | | leicht nach Moschus |
| 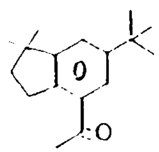 | $CH_2OH$<br>\|<br>CHOH<br>\|<br>$CH_2OH$ | 2<br>3 | $CH_2OH$<br>OH | C | $140_{(0.65)}$ | | leicht nach Moschus, holzig |

b) <u>Acetale</u> 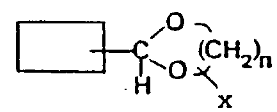

| | | | | | | |
|---|---|---|---|---|---|---|
| | $CH_2OHCH_2OH$ | 2 | H | C | 73-74 | nach Moschus, fruchtig |
| 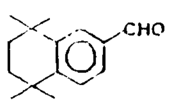 | $CH_2OHCHOHCH_3$ | 2 | $CH_3$ | C | 70-73 | nach Moschus, Kampher. |
| | $CH_2OHCH{=}CH{-}CH_2OH$ | $-CH_2CH_2{=}CHCH_2-$ | H | C | 75-76 | leicht nach Moschus, grün |
| 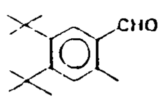 | $CH_2OHCH_2OH$ | 2 | H | C | 66-67 | leicht nach Moschus, fettig |

| | | | | | | | |
|---|---|---|---|---|---|---|---|
| 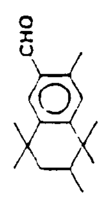 | $CH_2OHCH_2OH$ | 2 | H | C | | 72-73 | leicht nach Moschus |
| 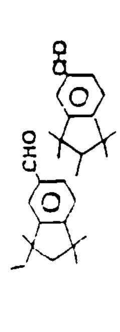 | $CH_2OHCH_2OH$ | 2 | H | C | $110_{(0.18)}$ | 50-58 | nach Moschus, grün, modrig |

Die Formeln für die cyclischen Acetale und Ketale (5-7 gliedrige Ringe) können im Prinzip wie folgt weiter aufgeschlüsselt werden:

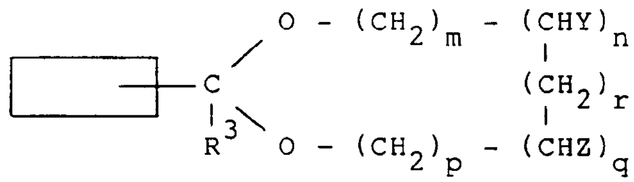

| | | | |
|---|---|---|---|
| $R^3$ | $= H, CH_3$ | m, n, p, q, r | = 0, 1 |
| Y, Z | $= CH_3..C_4$ | m + n + p + q + r | $= \leq 4$ |
| | $-CH_2OH.....C_4-OH$ | | |

Die neuen Acetale verbinden sich mit zahlreichen bekannten Riechstoffingredientien natürlichen oder synthetischen Ursprungs, wobei die Palette der natürlichen Rohstoffe sowohl leicht- als auch mittel- und schwerflüchtige Komponenten, und diejenige der Synthetika Vertreter aus Praktisch allen Stoffklassen umfassen kann, wie dies aus der folgenden Zusammenstellung ersichtlich ist:

- Naturprodukte, wie Baummoos-Absolue, Basilikumöl, Agrumenöle (wie Bergamotteöl, Mandarinenöl, etc.). Mastix-Absolue, Myrtenöl, Palmarosaöl, Patchouliöl, Petitgrainöl Paraguay, Wermutöl.
- Alkohole, wie Farnesol, Geraniol, Linalool, Nerol, Phenyläthylalkohol, Rhodinol, Zimtalkohol,
- Aldehyde, wie Citral, Helional®, α-Hexylzimtaldehyd, Hydroxycitronellal, Lilial® (p-tert.-Butyl-α-methyl-dihydrozimtaldehyd), Methylnonylacetaldehyd,
- Ketone, wie Allyljonon, α-Jonon, β-Jonon, Isoraldein (Isomethyl-α-ionon), Methyljonon,
- Ester, wie Allyl-phenoxyacetat, Benzyl-salicylat, Cinnamylpropionat, Citronellyl-acetat, Citronellyl-äthoxolat (Citronellyl) . O -CO -CO . $OC_2H_5$), Decylacetat, Dimethylbenzylcarbinyl-acetat, Dimethylbenzylcarbinyl-butyrat, Aethyl-acetoacetat, Aethyl-acetylacetat, Hexenyl-isobutyrat, Linalylacetat, Methyl-dihydrojasmonat, Styrallylacetat, Vetiverylacetat, etc.
- Lactone, wie γ-Undecalacton,
- verschiedene, in der Parfümerie oft benützte Komponenten, wie Ketonmoschus, Indol, p-Menthan-8-thiol-3-on, Methyleugenol.

Die Acetale lassen sich in weiten Grenzen einsetzen, die beispielsweise von ca. 0,1 (Detergentien) ca. - 20% (alkoholische Lösungen) in Kompositionen reichen können, ohne dass diese Werte jedoch Grenzwerte darstellen sollen, da der erfahrene Parfümeur auch mit noch geringeren Konzentrationen Effekte erzielen oder aber mit noch höheren Dosierungen neuartige Komplexe aufbauen kann. Die bevorzugten Konzentrationen bewegen sich zwischen ca. 1 und ca. 10%. Die mit I hergestellten Kompositionen lassen sich für alle Arten von parfümierten Verbrauchsgütern einsetzen (Eaux de Cologne, Eaux de Toilette, Extraits, Lotionen, Crèmes, Shampoos, Seifen, Salben, Puder, Zahnpasten, Mundwässer, Desodorantien, Detergentien, Gewebeveredler, Tabak, etc.).

Die Acetale können demgemäss bei der Herstellung von Kompositionen und - wie obige Zusammenstellung zeigt - unter Verwendung einer breiten Palette bekannter Riechstoffe bzw. Riechstoffgemische verwendet werden. Bei der Herstellung solcher Kompositionen können die oben aufgeführten bekannten Riechstoffe nach (dem Parfümeur bekannter) Art und Weise verwendet werden, wie z.B. aus W.A. Poucher, Perfumes, Cosmetics and Soaps 2, 7. Auflage, Chapman und Hall, London, 1974 hervorgehend.

Riechstoffkomposition

| | Gewichtsteile | Gewichtsteile |
|---|---|---|
| Bergamotte Ess. | 250,00 | 250,00 |
| Citronen Ess. | 150,00 | 150,00 |
| Lavandin Ess. | 100,00 | 100,00 |
| Cetone alpha (α-Isomethylionon) | 50,00 | 50,00 |
| Hedione (Methyldihydrojasmonat) | 50,00 | 50,00 |
| Sandalore | 20,00 | 20,00 |
| Fixolide | 40,00 | 40,00 |
| Coumarin krist. | 20,00 | 20,00 |
| Muskatnussessenz | 20,00 | 20,00 |
| Nelkenessenz | 20,00 | 20,00 |
| Estragon Ess. | 5,00 | 5,00 |
| Allylamylglycolat | 2,00 | 2,00 |
| LRG 1201 Evernyl (Methyl-2,4-dihydroxy-3,6-dimethylbenzoat) | 5,00 | 5,00 |
| Geranylacetat rein | 50,00 | 50,00 |
| Benzylacetat extra | 40,00 | 40,00 |
| I | | 70,00 |
| Pêche pur (Gamma-undecalacton) | 2,00 | 2,00 |
| Isoeugenol | 5,00 | 5,00 |
| Methylanthranilat extra | 1,00 | 1,00 |
| Basilikumessenz | 10,00 | 10,00 |
| Carbitol | 160,00 | 90,00 |
| | 1000,00 | 1000,00 |

Die Zugabe von 70%o (d.h. per 1000) I (2.4-Dimethyl-2-[1,1,4,4-tetramethyl-tetralin-6-yl]-1,3-dioxolan) verleiht der Komposition eine elegante Holznote, begleitet von einem warmen ambrigen Unterton. Gleichzeitig werden die Citrusnoten in angenehmer Weise unterstrichen. Die Komposition erhält ungleich mehr Frische und Volumen.

Die im Vordergrund des Interesses stehende Wäscheparfümierung kann wie folgt dokumentiert werden:

Zu diesem Zweck wurde ein konventioneller Wäscheveredler, nämlich ein Wäscheweichspüler, z.B. auf der üblichen Basis kationaktiver oder pseudokationaktiver Substanzen, z.B. vom quaternären Ammoniumtyp, mit 0,3% (w/w) Riechstoffgemisch in üblicher Weise parfümiert und nach dem Wäschevorgang (60°C) für den letzten Spülvorgang in einer Menge von wenigen g/l Wasser, z.B. ca. 3-6 g, im speziellen Fall 4,5 g/l zugegeben.

Als Standard wurde mit einem Riechstoffgemisch von 1,3,4,6,7,8-Hexahydro-4,6,6,7,8,8-hexamethyl-cyclopenta-γ-2-benzopyran ("Galaxolide")/1,1,2,4,4,7-hexamethyl-6-acetyl-1,2,3,4-tetrahydronaphthalin ("Fixolide") verglichen.

Tabelle III Waschversuche im Vergleich (konventionelle Textilveredler parfümiert mit 0,3% Riechstoffgemisch)

| Ref | Moschus/ oder Acetal Ketal (alle flüssig) % w/w | Bewertung Wäsche nass | | Bewertung Wäsche trocken |
|---|---|---|---|---|
| A | GALAXOLIDE / FIXOLIDE [Standard] 50 : 50 | - rel. wenig intensiv<br>- Moschusnote tritt kaum hervor | | - eher unangenehm<br>- kaum Moschus, fettig |
| B | GALAXOLIDE/ 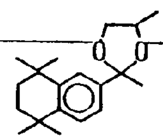 50 : 50 | stärker als alles andere, betont animalische Holznote + Costusaspekt | | stärker als alles andere, harmonischer Holzakkord mit animalischen und Patchouli-noten |
| C | FIXOLIDE/ 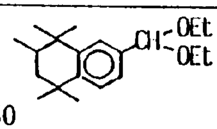 50 : 50 | mittel-starke Moschus-note | + erdige Aspekte | - angenehme Moschusnote mit hicht holzigem Aspekt |
| D | FIXOLIDE/ 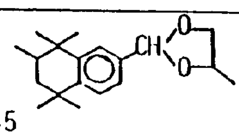 55 : 45 | | | |
| E | FIXOLIDE/ 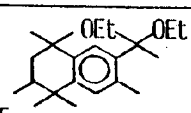 45 : 55 | | + Vanilla und Nitro-Moschusaspekte | - "saubere" Moschusnote mit guter Haftfestigkeit. |

## Patentansprüche

### Patentansprüche für folgende Vertragsstaaten : CH, DE, FR, GB, IT, LI, NL

1. Verbindungen der Formel

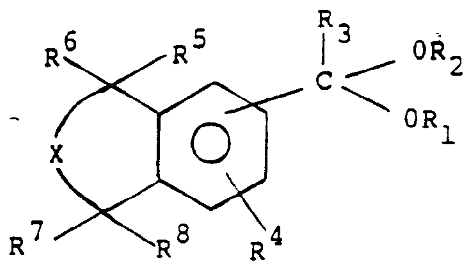

I

worin bedeuten:

| | |
|---|---|
| $R^1$, $R^2$ | $C_{1-4}$-Alkyl, $C_{2-4}$-Alkenyl oder |
| $R^1$ + $R^2$ | $C_{2-4}$-Alkylen, gegebenenfalls $C_{1-4}$-Alkyl-, $C_{2-4}$-Alkenyl- oder hydroxy-$C_{1-4}$alkyl- bzw. hydroxy-$C_{2-4}$-alkenyl-substituiert |
| $R^3$ | H, $CH_3$ |
| $R^4$ | H, $C_{1-4}$-Alkyl |
| $R^5$, $R^6$, $R^7$,$R^8$ | H, $CH_3$, $C_2H_5$, $CH_2CH_2CH_3$, CH $(CH_3)_2$, |
| X | Methylen, Aethyliden, Propyliden, Aethylen, Propylen, 2,3-Butyliden |

Gesamtzahl der Kohlenstoffatome von $R^1$, $R^2$ $\leqslant$ 6

Gesamtzahl der Kohlenstoffatome von $R^1$, $R^2$, $R^3$. $R^4$ $\leqslant$ 11

Gesamtzahl der Kohlenstoffatome von $R^5$, $R^6$, $R^7$, $R^8$ $<$ 6

mit der Ausnahme derjenigen Verbindungen I, worin

| | |
|---|---|
| $R^3$ | H |
| $R^4$ | H oder $CH_3$ |
| $R^5$, $R^6$, $R^7$, $R^8$ | $CH_3$ |
| X | Aethylen und |
| $R^1$, $R^2$ | $C_{1-4}$-Alkyl |

2. Die offenkettigen Acetale und Ketale der Oxo-tetraline der Formel I gemäss Anspruch 1.

3. Die offenkettigen Acetale und Ketale der Oxo-indane der Formel I gemäss Anspruch 1.

4. Die cyclischen Acetale und Ketale der Oxo-indane der Formel I gemäss Anspruch 1.

5. Die cyclischen Acetale und Ketale der Oxo-tetraline der Formel I gemäss Anspruch 1.

6. 2,4-Dimethyl-2-[1,1,4,4-tetramethyl-tetralin-6-yl]-1,3-dioxolan.

7. 2-Methyl-4 ethyl-2-[1,1,4,4-tetramethyl-tetralin-6-yl]-1,3-dioxolan.

8. 7-[Formyl-diethyl-acetal]-1,1,2,4,4,-pentamethyl-tetralin.

**9.** 2,4-Dimethyl-2-[1,1,4,4-tetramethyl-tetralin-6-yl]-1,3-dioxan.

**10.** 6-(Acetyl-diethyl-ketal)-1,1,2,4,4,7-hexamethyl-tetralin.

**11.** 2,4-Dimethyl-2-[1,1,2,4,4,7-hexamethyl-tetralin-6-yl]-1,3-dioxolan.

**12.** 2,4-Dimethyl-2-[1,1,2,4,4,7-hexamethyl-tetralin-6-yl]-1,3-dioxolan.

**13.** Riechstoffkomposition, gekennzeichnet durch einen Gehalt an einer Verbindung der Formel I gemäss Anspruch 1.

**14.** Riechstoffkomposition, gekennzeichnet durch einen Gehalt an 2,4-Dimethyl-2-[1,1,4,4-tetramethyl-tetralin-6-yl]-1,3-dioxolan.

**15.** Riechstoffkomposition, gekennzeichnet durch einen Gehalt an 7-(Formyl-diethyl-acetal]-1,1,2,4,4,-pentamethyl-tetralin.

**16.** Verfahren zur Herstellung der Verbindungen

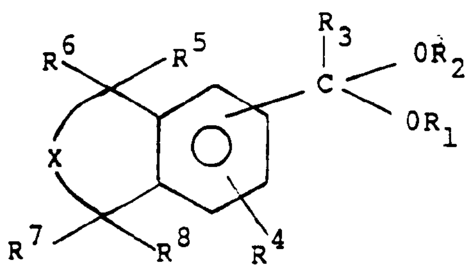

I

worin bedeuten:

| | |
|---|---|
| $R^1$, $R^2$ | $C_{1-4}$-Alkyl, $C_{2-4}$-Alkenyl oder |
| $R^1$ + $R^2$ | $C_{2-4}$-Alkylen, gegebenenfalls $C_{1-4}$-alkyl-, $C_{2-4}$-alkenyl- oder hydroxy-$C_{1-4}$-alkyl- bzw. hydroxy-$C_{2-4}$-alkenyl-substituiert |
| $R^3$ | H, $CH_3$ |
| $R^4$ | H, $C_{1-4}$-Alkyl |
| $R^5$, $R^6$, $R^7$, $R^8$, | H, $CH_3$, $C_2H_5$, $CH_2CH_2CH_3$, $CH(CH_3)_2$ |
| X | Methylen, Aethyliden, Propyliden, Aethylen, Propylen, 2,3-Butyliden |

Gesamtzahl der Kohlenstoffatome von $R^1$, $R^2$ $\leq 6$

Gesamtzahl der Kohlenstoffatome von $R^1$, $R^2$, $R^3$. $R^4$ $\leq 11$

Gesamtzahl der Kohlenstoffatome von $R^5$, $R^6$, $R^7$, $R^8$ $\leq 6$

mit der Ausnahme derjenigen Verbindungen I, worin

| | |
|---|---|
| $R^3$ | H |
| $R^4$ | H oder $CH_3$ |
| $R^5$, $R^6$, $R^7$, $R^8$ | $CH_3$ |
| X | Aethylen und |
| $R^1$, $R^2$ | $C_{1-4}$-Alkyl |

dadurch gekennzeichnet, dass man eine Verbindung der Formel

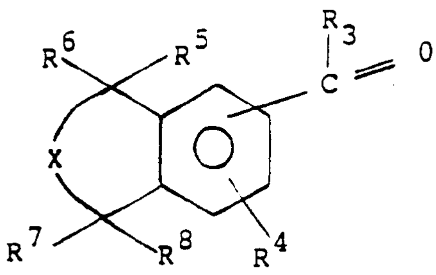

II

in an sich bekannter Weise acetalisiert bzw. ketalisiert,
oder eine Verbindung I transacetalisiert bzw. -ketalisiert,
oder eine Verbindung der Formel

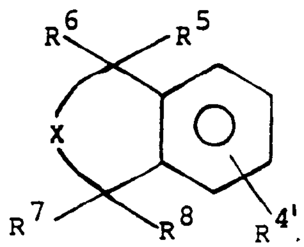

III

worin

| | |
|---|---|
| $R^{4'}$ | $CH_3$, $C_2H_5$ und |
| $R^5$, $R^6$, $R^7$, $R^8$ | obige Bedeutung besitzen, |

in Gegenwart eines $C_{1-3}$ Alkanols $R^{2'}OH$ und eines Leitsalzes elektrochemisch oxidiert.

**17.** Verwendung einer Verbindung der Formel I gemäss Anspruch 1 als Riechstoff.

**18.** Verwendung von 2,4-Dimethyl-2-[1,1,4,4-tetramethyl-tetralin-6-yl]-1,3-dioxolan als Riechstoff.

**19.** Verwendung von 7-[Formyl-diethyl-acetal]-1,1,2,4,4-pentamethyl-tetralin als Riechstoff.

**Patentansprüche für folgenden Vertragsstaat : ES**

**1.** Verfahren zur Herstellung der Verbindungen

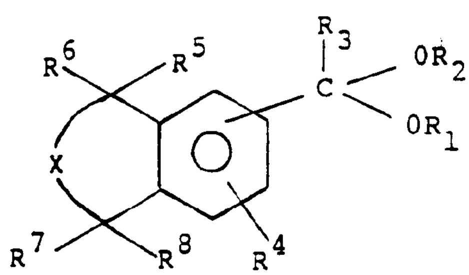

I

worin bedeuten:

| | |
|---|---|
| $R^1$, $R^2$ | $C_{1-4}$-Alkyl, $C_{2-4}$-Alkenyl oder |
| $R^1 + R^2$ | $C_{2-4}$-Alkylen, gegebenenfalls $C_{1-4}$-alkyl-, $C_{2-4}$-alkenyl- oder hydroxy-$C_{1-4}$-alkyl- bzw. hydroxy-$C_{2-4}$-alkenyl-substituiert |
| $R^3$ | H, $CH_3$ |
| $R^4$ | H, $C_{1-4}$-Alkyl |
| $R^5$, $R^6$, $R^7$, $R^8$, | H, $CH_3$, $C_2H_5$, $CH_2CH_2CH_3$, $CH(CH_3)_2$ |
| X | Methylen, Aethyliden, Propyliden, Aethylen, Propylen, 2,3-Butyliden |

Gesamtzahl der Kohlenstoffatome von $R^1$, $R^2$ $\leq$ 6
Gesamtzahl der Kohlenstoffatome von $R^1$, $R^2$, $R^3$. $R^4$ $\leq$ 11
Gesamtzahl der Kohlenstoffatome von $R^5$, $R^6$, $R^7$, $R^8$ $\leq$ 6

mit der Ausnahme derjenigen Verbindungen I, worin

| | |
|---|---|
| $R^3$ | H |
| $R^4$ | H oder $CH_3$ |
| $R^5$, $R^6$, $R^7$, $R^8$ | $CH_3$ |
| X | Aethylen |
| | und |
| $R^1$, $R^2$ | $C_{1-4}$-Alkyl |

dadurch gekennzeichnet, dass man eine Verbindung der Formel

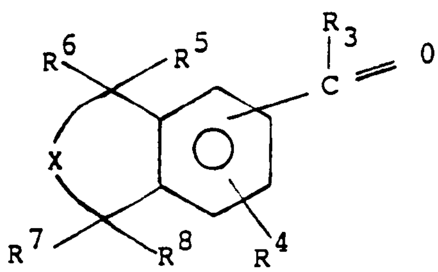

II

in an sich bekannter Weise acetalisiert bzw. ketalisiert,
oder eine Verbindung I transacetalisiert bzw. -ketalisiert,
oder eine Verbindung der Formel

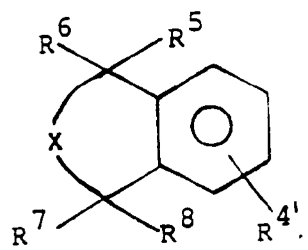

III

worin

| | |
|---|---|
| $R^{4'}$ | $CH_3$, $C_2H_5$ und |
| $R^5$, $R^6$, $R^7$, $R^8$ | obige Bedeutung besitzen, |

in Gegenwart eines $C_{1-3}$ Alkanols $R^{2'}$ OH und eines Leitsalzes elektrochemisch oxidiert.

2. Verfahren nach Anspruch 1, dadurch gekennzeichnet, dass die Verbindung der Formel I 2,4-Dimethyl-2-[1,1,4,4-tetramethyl-tetralin-6-yl]-1,3-dioxolan ist.

3. Verfahren nach Anspruch 1, dadurch gekennzeichnet, dass die Verbindung der Formel I 7-[Formyl-diethylacetal]-1,1,2,4,4,-pentamethyl-tetralin ist.

4. Riechstoffkomposition, gekennzeichnet durch einen Gehalt an einer Verbindung der Formel

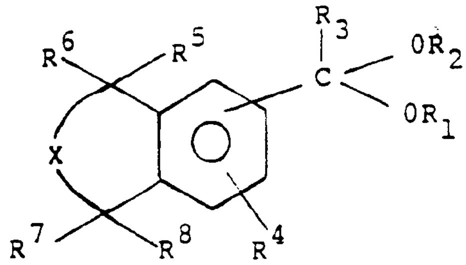

I

worin bedeuten:

| | |
|---|---|
| $R^1$, $R^2$ | $C_{1-4}$-Alkyl, $C_{2-4}$-Alkenyl |
| | oder |
| $R^1$ + $R^2$ | $C_{2-4}$-Alkylen, gegebenenfalls $C_{1-4}$-alkyl-, $C_{2-4}$-alkenyl- oder hydroxy-$C_{1-4}$alkyl- bzw. hydroxy-$C_{2-4}$-alkenyl-substituiert |
| $R^3$ | H, $CH_3$ |
| $R^4$ | H, $C_{1-4}$-Alkyl |
| $R^5$, $R^6$, $R^7$,$R^8$ | H, $CH_3$,$C_2H_5$, $CH_2CH_2CH_3$, CH $(CH_3)_2$, |
| X | Methylen, Aethyliden, Propyliden, Aethylen, Propylen, 2,3-Butyliden |

| | |
|---|---|
| Gesamtzahl der Kohlenstoffatome von $R^1$, $R^2$ | $\leqslant$ 6 |
| Gesamtzahl der Kohlenstoffatome von $R^1$, $R^2$, $R^3$, $R^4$ | $\leqslant$ 11 |
| Gesamtzahl der Kohlenstoffatome von $R^5$, $R^6$, $R^7$, $R^8$ | $\leqslant$ 6 |

mit der Ausnahme derjenigen Verbindungen I, worin

| | |
|---|---|
| $R^3$ | H |
| $R^4$ | H oder $CH_3$ |
| $R^5$, $R^6$, $R^7$, $R^8$ | $CH_3$ |
| X | Aethylen |
| | und |
| $R^1$, $R^2$ | $C_{1-4}$-Alkyl |

5. Riechstoffkomposition, gekennzeichnet durch einen Gehalt an 2,4-Dimethyl-2-[1,1,4,4-tetramethyl-tetralin-6-yl]-1,3-dioxolan.

6. Riechstoffkomposition, gekennzeichnet durch einen Gehalt an 7-[Formyl-diethyl-acetal]-1,1,2,4,4,-pentamethyl-tetralin.

7. Verwendung von Verbindungen der Formel

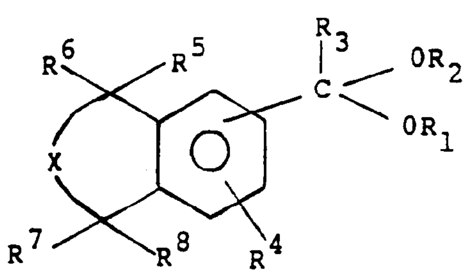

I

worin bedeuten:

| | |
|---|---|
| $R^1$, $R^2$ | $C_{1-4}$-Alkyl, $C_{2-4}$-Alkenyl |
| | oder |

| | |
|---|---|
| $R^1 + R^2$ | $C_{2-4}$-Alkylen, gegebenenfalls $C_{1-4}$-alkyl-, $C_{2}$ $_4$-alkenyl- oder hydroxy-$C_{1-4}$alkyl- bzw. hydroxy-$C_{2-4}$-alkenyl-substituiert |
| $R^3$ | H, $CH_3$ |
| $R^4$ | H, $C_{1-4}$-Alkyl |
| $R^5$, $R^6$, $R^7$,$R^8$ | H, $CH_3$,$C_2H_5$, $CH_2CH_2CH_3$, CH $(CH_3)_2$, |
| X | Methylen, Aethyliden, Propyliden, Aethylen, Propylen, 2,3-Butyliden |

| | | |
|---|---|---|
| Gesamtzahl der Kohlenstoffatome von | $R^1$, $R^2$ | $\leqslant$ 6 |
| Gesamtzahl der Kohlenstoffatome von | $R^1$, $R^2$, $R^3$. $R^4$ | $\leqslant$ 11 |
| Gesamtzahl der Kohlenstoffatome von | $R^5$, $R^6$, $R^7$, $R^8$ | $\leqslant$ 6 |

mit der Ausnahme derjenigen Verbindungen I, worin

| | |
|---|---|
| $R^3$ | H |
| $R^4$ | H oder $CH_3$ |
| $R^5$, $R^6$, $R^7$, $R^8$ | $CH_3$ |
| X | Aethylen |
| | und |
| $R^1$, $R^2$ | $C_{1-4}$-Alkyl |

**8.** Verwendung von 2,4-Dimethyl-2-[1,1,4,4-tetramethyl-tetralin-6-yl]-1,3-dioxolan als Riechstoff.

**9.** Verwendung von 7-[Formyl-diethyl-acetal]-1,1,2,4,4-pentamethyl-tetralin als Riechstoff.

## Claims

### Claims for the following Contracting States : CH, DE, FR, GB, IT, LI, NL

**1.** Compounds of the formula

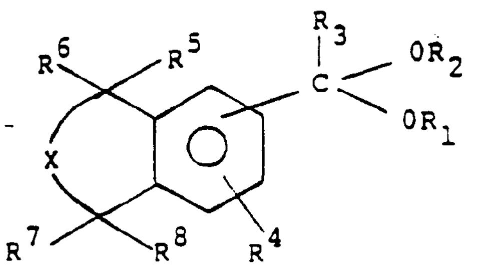

I

wherein

| | |
|---|---|
| $R^1$, $R^2$ signify | $C_{1-4}$-alkyl, $C_{2-4}$-alkenyl |
| | or |
| $R^1 + R^2$ signify | $C_{2-4}$-alkylene which may be $C_{1-4}$-alkyl-, $C_{2-4}$-alkenyl- or hydroxy-$C_{1-4}$-alkyl- or hydroxy-$C_{2-4}$-alkenyl-substituted |
| $R^3$ signifies | H, $CH_3$ |
| $R^4$ signifies | H, $C_{1-4}$-alkyl |
| $R^5$, $R^6$, $R^7$, $R^8$ signify | H, $CH_3$, $C_2H_5$, $CH_2CH_2CH_3$, $CH(CH_3)_2$ |
| X signifies | methylene, ethylidene, propylidene, ethylene, propylene, 2,3-butylidene |

total number of carbon atoms of $R^1$, $R^2$ $\leq$ 6

total number of carbon atoms of $R^1$, $R^2$, $R^3$, $R^4$ $\leq$ 11

total number of carbon atoms of $R^5$, $R^6$, $R^7$, $R^8$ $\leq$ 6,

with the exception of those compounds I in which

| | |
|---|---|
| $R^3$ is | H |
| $R^4$ is | H or $CH_3$ |
| $R^5$, $R^6$, $R^7$, $R^8$ are | $CH_3$ |
| X is | ethylene |
| | and |
| $R^1$, $R^2$ are | $C_{1-4}$alkyl |

**2.** The open-chain acetals and ketals of the oxo-tetralins of formula I in accordance with claim 1.

**3.** The open-chain acetals and ketals of the oxo-indanes of formula I in accordance with claim 1.

**4.** The cyclic acetals and ketals of the oxo-indanes of formula I in accordance with claim 1.

**5.** The cyclic acetals and ketals of the oxo-tetralins of formula I in accordance with claim 1.

**6.** 2,4-Dimethyl-2-[1,1,4,4-tetramethyl-tetralin-6-yl]-1,3-dioxolane.

**7.** 2-Methyl-4 ethyl-2-[1,1,4,4-tetramethyl-tetralin-6-yl]-1,3-dioxolane.

**8.** 7-[Formyl diethyl acetal]-1,1,2,4,4-pentamethyl-tetralin.

**9.** 2,4-Dimethyl-2-[1,1,4,4-tetramethyl-tetralin-6-yl]-1,3-dioxane.

**10.** 6-(Acetyl diethyl ketal)-1,1,2,4,4,7-hexamethyl-tetralin.

**11.** 2,4-Dimethyl-2-[1,1,2,4,4,7-hexamethyl-tetralin-6-yl]-1,3-dioxolane.

**12.** 2,4-Dimethyl-2-[1,1,2,4,4,7-hexamethyl-tetralin-6-yl]-1,3-dioxolane.

**13.** An odorant composition, characterized in that it contains a compound of formula I in accordance with claim 1.

**14.** An odorant composition, characterized in that it contains 2,4-dimethyl-2-[1,1,4,4-tetramethyl-tetralin-6-yl]-1,3-dioxolane.

**15.** An odorant composition, characterized in that it contains 7-[formyl diethyl acetal]-1,1,2,4,4-pentamethyl-tetralin.

**16.** A process for the manufacture of the compounds

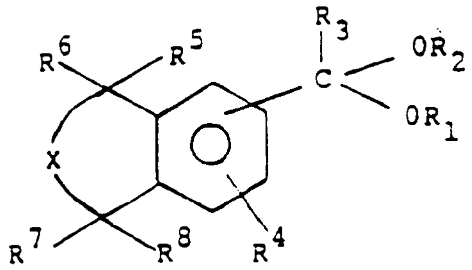

I

wherein

| | |
|---|---|
| $R^1$, $R^2$ signify | $C_{1-4}$-alkyl, $C_{2-4}$-alkenyl |
| | or |
| $R^1$ + $R^2$ signify | $C_{2-4}$-alkylene which may be $C_{1-4}$-alkyl-, $C_{2-4}$-alkenyl- or hydroxy-$C_{1-4}$-alkyl- or hydroxy-$C_{2-4}$-alkenyl-substituted |
| $R^3$ signifies | H, $CH_3$ |
| $R^4$ signifies | H, $C_{1-4}$-alkyl |
| $R^5$, $R^6$, $R^7$, $R^8$ signify | H, $CH_3$, $C_2H_5$, $CH_2CH_2CH_3$, $CH(CH_3)_2$ |
| X signifies | methylene, ethylidene, propylidene, ethylene, propylene, 2,3-butylidene |

total number of carbon atoms of $R^1$, $R^2$ $\leqslant 6$

total number of carbon atoms of $R^1$, $R^2$, $R^3$, $R^4$ $\leqslant 11$

total number of carbon atoms of $R^5$, $R^6$, $R^7$, $R^8$ $\leqslant 6$,

with the exception of those compounds I in which

| | |
|---|---|
| $R^3$ is | H |
| $R^4$ is | H or $CH_3$ |
| $R^5$, $R^6$, $R^7$, $R^8$ are | $CH_3$ |
| X is | ethylene |
| | and |
| $R^1$, $R^2$ are | $C_{1-4}$alkyl |

characterized by acetalizing or ketalizing a compound of the formula

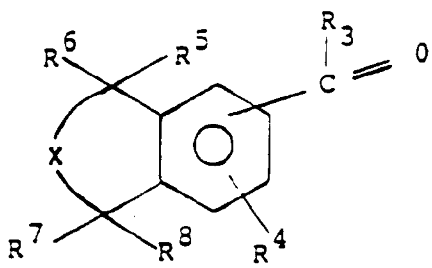

II

in a manner known per se, or transacetalizing or transketalizing a compound I, or electrochemically oxidizing a compound of the formula

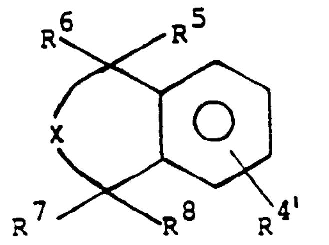

III

wherein

| | |
|---|---|
| $R^{4'}$ signifies | $CH_3$, $C_2H_5$ and |
| $R^5$, $R^6$, $R^7$, $R^8$ have | the above significance, |

in the presence of a $C_{1-3}$alkanol $R^{2'}$ OH and a conducting salt.

17. The use of a compound of formula I in accordance with claim 1 as an odorant.

18. The use of 2,4-dimethyl-2-[1,1,4,4-tetramethyl-tetralin-6-yl]-1,3-dioxolane as an odorant.

19. The use of 7-[formyl diethyl acetal]-1,1,2,4,4-pentamethyl-tetralin as an odorant.

**Claims for the following Contracting State : ES**

1. A process for the manufacture of the compounds

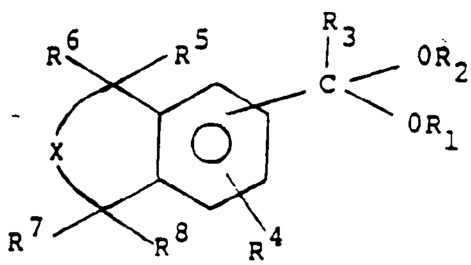

I

wherein

| | |
|---|---|
| $R^1$, $R^2$ signify | $C_{1-4}$-alkyl, $C_{2-4}$-alkenyl |
| | or |
| $R^1$ + $R^2$ signify | $C_{2-4}$-alkylene which may be $C_{1-4}$-alkyl-, $C_{2-4}$-alkenyl- or hydroxy-$C_{1-4}$-alkyl- or hydroxy-$C_{2-4}$-alkenyl-substituted |
| $R^3$ signifies | H, $CH_3$ |
| $R^4$ signifies | H, $C_{1-4}$-alkyl |
| $R^5$, $R^6$, $R^7$, $R^8$ signify | H, $CH_3$, $C_2H_5$, $CH_2CH_2CH_3$, $CH(CH_3)_2$ |
| X signifies | methylene, ethylidene, propylidene, ethylene, propylene, 2,3-butylidene |

| | |
|---|---|
| total number of carbon atoms of $R^1$, $R^2$ | $\leqslant$ 6 |
| total number of carbon atoms of $R^1$, $R^2$, $R^3$, $R^4$ | $\leqslant$ 11 |
| total number of carbon atoms of $R^5$, $R^6$, $R^7$, $R^8$ | $\leqslant$ 6, |

with the exception of those compounds I in which

| | |
|---|---|
| $R^3$ is | H |
| $R^4$ is | H or $CH_3$ |
| $R^5$, $R^6$, $R^7$, $R^8$ are | $CH_3$ |

| | |
|---|---|
| X is | ethylene and |
| $R^1$, $R^2$ are | $C_{1-4}$alkyl, |

characterized by acetalizing or ketalizing a compound of the formula

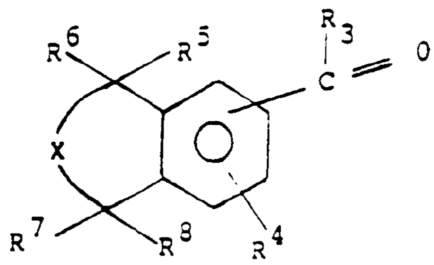

II

in a manner known per se, or transacetalizing or transketalizing a compound I, or electrochemically oxidizing a compound of the formula

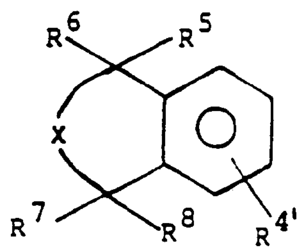

III

wherein

| | |
|---|---|
| $R^{4'}$ signifies | $CH_3$, $C_2H_5$ and |
| $R^5$, $R^6$, $R^7$, $R^8$ have | the above significance, |

in the presence of a $C_{1-3}$alkanol $R^{2'}$ OH and a conducting salt.

2. A process according to claim 1, characterized in that the compound of formula I is 2,4-dimethyl-2-[1,1,4,4-tetramethyl-tetralin-6-yl]-1,3-dioxolane.

3. A process according to claim 1, characterized in that the compound of formula I is 7-[formyl diethyl acetal]-1,1,2,4,4-pentamethyl-tetralin.

4. An odorant composition, characterized in that it contains a compound of the formula

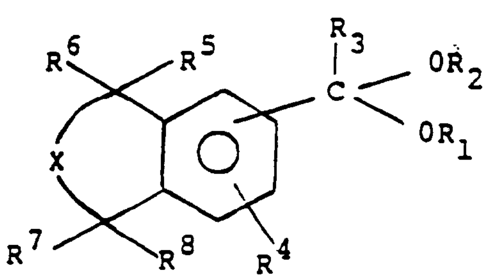

I

wherein

| | |
|---|---|
| $R^1$, $R^2$ signify | $C_{1-4}$-alkyl, $C_{2-4}$-alkenyl or |
| $R^1$ + $R^2$ signify | $C_{2-4}$-alkylene which may be $C_{1-4}$-alkyl-, $C_{2-4}$-alkenyl- or hydroxy-$C_{1-4}$-alkyl- or hydroxy-$C_{2-4}$-alkenyl-substituted |
| $R^3$ signifies | H, $CH_3$ |
| $R^4$ signifies | H, $C_{1-4}$-alkyl |

| | |
|---|---|
| $R^5$, $R^6$, $R^7$, $R^8$ signify | H, $CH_3$, $C_2H_5$, $CH_2CH_2CH_3$, $CH(CH_3)_2$ |
| X signifies | methylene, ethylidene, propylidene, ethylene, propylene, 2,3-butylidene |

total number of carbon atoms of $R^1$, $R^2$ $\leqslant$ 6

total number of carbon atoms of $R^1$, $R^2$, $R^3$, $R^4$ $\leqslant$ 11

total number of carbon atoms of $R^5$, $R^6$, $R^7$, $R^8$ $\leqslant$ 6,

with the exception of those compounds I in which

| | |
|---|---|
| $R^3$ is | H |
| $R^4$ is | H or $CH_3$ |
| $R^5$, $R^6$, $R^7$, $R^8$ are | $CH_3$ |
| X is | ethylene |
| | and |
| $R^1$, $R^2$ are | $C_{1-4}$alkyl |

5. An odorant composition, characterized in that it contains 2,4-dimethyl-2-[1,1,4,4-tetramethyl-tetralin-6-yl]-1,3-dioxolane.

6. An odorant composition, characterized in that it contains 7-[formyl diethyl acetal]-1,1,2,4,4-pentamethyl-tetralin.

7. The use of compounds of the formula

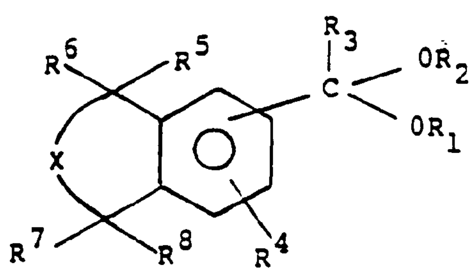

I

wherein

| | |
|---|---|
| $R^1$, $R^2$ signify | $C_{1-4}$-alkyl, $C_{2-4}$-alkenyl |
| | or |
| $R^1$ + $R^2$ signify | $C_{2-4}$-alkylene which may be $C_{1-4}$-alkyl-, $C_{2-4}$-alkenyl- or hydroxy-$C_{1-4}$-alkyl- or hydroxy-$C_{2-4}$-alkenyl-substituted |
| $R^3$ signifies | H, $CH_3$ |
| $R^4$ signifies | H, $C_{1-4}$-alkyl |
| $R^5$, $R^6$, $R^7$, $R^8$ signify | H, $CH_3$, $C_2H_5$, $CH_2CH_2CH_3$, $CH(CH_3)_2$ |
| X signifies | methylene, ethylidene, propylidene, ethylene, propylene, 2,3-butylidene |

total number of carbon atoms of $R^1$, $R^2$ $\leqslant$ 6

total number of carbon atoms of $R^1$, $R^2$, $R^3$, $R^4$ $\leqslant$ 11

total number of carbon atoms of $R^5$, $R^6$, $R^7$, $R^8$ $\leqslant$ 6,

with the exception of those compounds I in which

| | |
|---|---|
| $R^3$ is | H |
| $R^4$ is | H or $CH_3$ |
| $R^5$, $R^6$, $R^7$, $R^8$ are | $CH_3$ |
| X is | ethylene |
| | and |
| $R^1$, $R^2$ are | $C_{1-4}$alkyl |

as odorants.

8. The use of 2,4-dimethyl-2-[1,1,4,4-tetramethyl-tetralin-6-yl]-1,3-dioxolane as an odorant.

9. The use of 7-[formyl diethyl acetal]-1,1,2,4,4-pentamethyl-tetralin as an odorant.

## Revendications

### Revendications pour les Etats contractants suivants : CH, DE, FR, GB, IT, LI, NL

1. Composés de formule

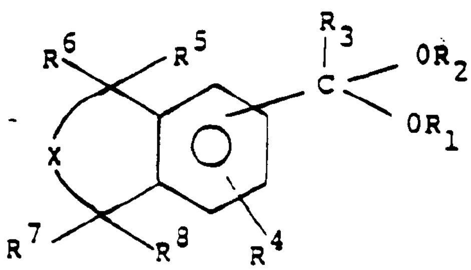

I

dans laquelle

| | |
|---|---|
| $R^1$, $R^2$ | représentent des groupes alkyle en C 1-C 4, alcényle en C 2-C 4, ou bien |
| $R^1$+$R^2$ | représentent un groupe alkylène en C 2-C 4 éventuellement substitué par des groupes alkyle en C 1-C 4, alcényle en C 2-C 4 ou hydroxyalkyle en C 1-C 4 ou hydroxyalcényle en C 2-C 4, |
| $R^3$ | représente H, $CH_3$, |
| $R^4$ | représente H, un groupe alkyle en C 1-C 4, |
| $R^5$, $R^6$, $R^7$, $R^8$ | représentent H, $CH_3$, $C_2H_5$, $CH_2CH_2CH_3$, $CH(CH_3)_2$, |
| X | représente un groupe méthylène, éthylidène, propylidène, éthylène, propylène, 2,3-butylidène, |

le nombre total des atomes de carbone de $R^1$, $R^2$ est inférieur ou égal à 6,
le nombre total des atomes de carbone de $R^1$, $R^2$, $R^3$, $R^4$ est inférieur ou égal à 11,
le nombre total des atomes de carbone de $R^5$, $R^6$, $R^7$, $R^8$ est inférieur ou égal à 6,
à l'exclusion des composés I pour lesquels

| | |
|---|---|
| $R^3$ | représente H |
| $R^4$ | représente H ou $CH_3$ |
| $R^5$, $R^6$, $R^7$, $R^8$ | représentent $CH_3$, |
| X | représente un groupe éthylène, et |
| $R^1$, $R^2$ | représentent des groupes alkyle en C 1-C 4. |

2. Les acétals acycliques des oxotétralines de formule I de la revendication 1.

3. Les acétals acycliques des oxo-indanes de formule I de la revendication 1.

4. Les acétals cycliques des oxo-indanes de formule I de la revendication 1.

5. Les acétals cycliques des oxo-tétralines de formule I de la revendication 1.

6. Le 2,4-diméthyl-2-(1,1,4,4-tétraméthyl-tétraline-6-yl)-1,3-dioxolanne.

7. Le 2-méthyl-4-éthyl-2-(1,1,4,4-tétraméthyl-tétraline-6-yl)-1,3-dioxolanne.

8. La 7-(formyl-diéthyl-acétal)-1,1,2,4,4-pentaméthyl-tétraline.

9. Le 2,4-diméthyl-2-(1,1,4,4-tétraméthyl-tétraline-6-yl)-1,3-dioxanne.

10. La 6-(acétyl-diéthyl-acétal)-1,1,2,4,4,7-hexaméthyl-tétraline.

11. Le 2,4-diméthyl-2-(1,1,2,4,4,7-hexaméthyl-tétraline-6-yl)-1,3-dioxolanne.

12. Le 2,4-diméthyl-2-(1,1,2,4,4,7-hexaméthyl-tétraline-6-yl)-1,3-dioxolanne.

13. Composition de parfum, caractérisée en ce qu'elle contient un composé de formule I de la revendication 1.

14. Composition de parfum, caractérisée en ce qu'elle contient du 2,4-diméthyl-2-(1,1,4,4-tétraméthyl-tétraline-6-yl)-1,3-dioxolanne.

15. Composition de parfum, caractérisée en ce qu'elle contient de la 7-(formyl-diéthylacétal)-1,1,2,4,4-pentaméthyl-tétraline.

16. Procédé de préparation des composés

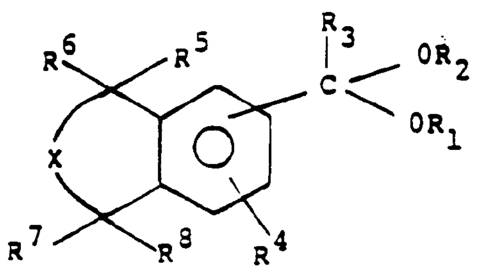

I

dans laquelle

| | |
|---|---|
| $R^1$, $R^2$ | représentent des groupes alkyle en C 1-C 4, alcényle en C 2-C 4, ou bien |
| $R^1 + R^2$ | représentent un groupe alkylène en C 2-C 4 éventuellement substitué par des groupes alkyle en C 1-C 4, alcényle en C 2-C 4 ou hydroxyalkyle en C 1-C 4 ou hydroxyalcényle en C 2-C 4, |
| $R^3$ | représente H ou $CH_3$, |
| $R^4$ | représente H, un groupe alkyle en C 1-C 4, |
| $R^5$, $R^6$, $R^7$, $R^8$ | représentent H, $CH_3$, $C_2H_5$, $CH_2CH_2CH_3$, $CH(CH_3)_2$, |
| X | représente un groupe méthylène, éthylidène, propylidène, éthylène, propylène, 2,3-butylidène, |

le nombre total des atomes de carbone de $R^1$, $R^2$ est inférieur ou égal à 6,
le nombre total des atomes de carbone de $R^1$, $R^2$, $R^3$, $R^4$ est inférieur ou égal à 11,
le nombre total des atomes de carbone de $R^5$, $R^6$, $R^7$, $R^8$ est inférieur ou égal à 6,
à l'exclusion des composés I pour lesquels

| | |
|---|---|
| $R^3$ | représente H, |
| $R^4$ | représente H ou $CH_3$, |
| $R^5$, $R^6$, $R^7$, $R^8$ | représentent $CH_3$, |
| X | représente un groupe éthylène, et |
| $R^1$, $R^2$ | représentent des groupes alkyle en C 1-C 4, |

caractérisé en ce que l'on acétalise de manière connue en soi un composé de formule

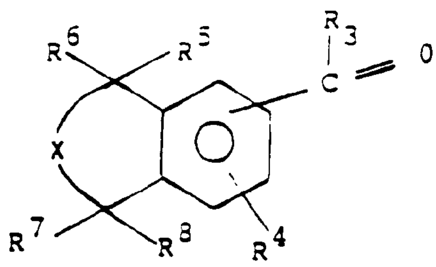

II

ou bien on transacétalise un composé I,
ou bien on soumet un composé de formule

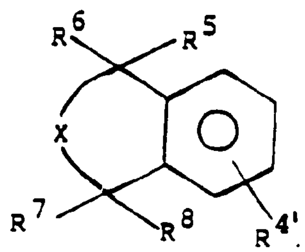

III

dans laquelle

| | |
|---|---|
| $R^{4'}$ | représente $CH_3$, $C_2H_5$ et |
| $R^5$, $R^6$, $R^7$, $R^8$ | ont les significations indiquées ci-dessus, |

à oxydation électrochimique en présence d'un alcanol $R^{2'}$ OH en C 1-C 3 et d'un sel conducteur.

**17.** Utilisation d'un composé de formule I de la revendication 1 en tant que matière odorante.

**18.** Utilisation du 2,4-diméthyl-2-(1,1,4,4-tétraméthyl-tétraline-6-yl)-1,3-dioxolanne en tant que matière odorante.

**19.** utilisation de la 7-(formyl-diéthyl-acétal)-1,1,2,4,4-pentaméthyl-tétraline en tant que matière odorante.

**Revendications pour l'Etat contractant suivant : ES**

**1.** Procédé de préparation des composés

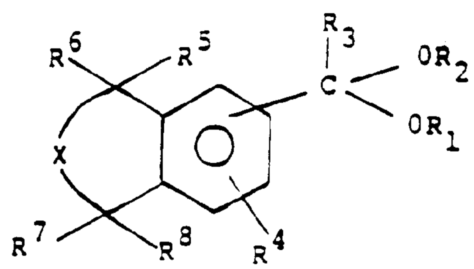

I

dans lesquels

| | |
|---|---|
| $R^1$, $R^2$ | représentent des groupes alkyle en C 1-C 4, alcényle en C 2-C 4, ou bien |
| $R^1$ + $R^2$ | représentent un groupe alkylène en C 2-C 4 éventuellement substitué par des groupes alkyle en C 1-C 4, alcényle en C 2-C 4, hydroxyalkyle en C 1-C 4, hydroxyalcényle en C 2-C 4, |
| $R^3$ | représente H, $CH_3$, |
| $R^4$ | représente H, un groupe alkyle en C 1-C 4, |
| $R^5$, $R^6$, $R^7$, $R^8$ | représentent H, $CH_3$, $C_2H_5$, $CH_2CH_2CH_3$, $CH(CH_3)_2$, |

X représente un groupe méthylène, éthylidène, propylidène, éthylène, propylène, 2,3-butylidène,

le nombre total des atomes de carbone de $R^1$, $R^2$ est inférieur ou égal à 6,

le nombre total des atomes de carbone de $R^1$, $R^2$, $R^3$, $R^4$ est inférieur ou égal à 11,

le nombre total des atomes de carbone de $R^5$, $R^6$, $R^7$, $R^8$ est inférieur ou égal à 6,

à l'exclusion des composés pour lesquels

$R^3$ représente H,

$R^4$ représente H ou $CH_3$,

$R^5$, $R^6$, $R^7$, $R^8$ représentent $CH_3$,

X représente un groupe éthylène, et

$R^1$, $R^2$ représentent des groupes alkyle en C 1-C 4,

caractérisé en ce que l'on acétalise de manière connue en soi un composé de formule

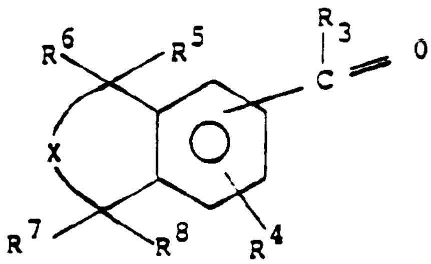

II

ou bien on transacétalise un composé I,

ou bien on soumet un composé de formule

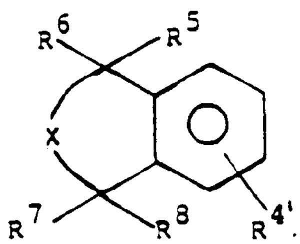

III

dans laquelle

$R^{4'}$ représente $CH_3$, $C_2C_5$, et

$R^5$, $R^6$, $R^7$, $R^8$ ont les significations indiquées ci-dessus,

à oxydation électrochimique en présence d'un alcanol $R^{2'}$ OH en C 1-C 3 et d'un sel conducteur.

2. Procédé selon la revendication 1, caractérisé en ce que le composé de formule I est le 2,4-diméthyl-2-(1,1,4,4-tétraméthyl-tétraline-6-yl)-1,3-dioxolanne.

3. Procédé selon la revendication 1, caractérisé en ce que le composé de formule I est la 7-(formyl-diéthyl-acétal)-1,1,2,4,4-pentaméthyl-tétraline.

4. Composition de parfum, caractérisée en ce qu'elle contient un composé de formule

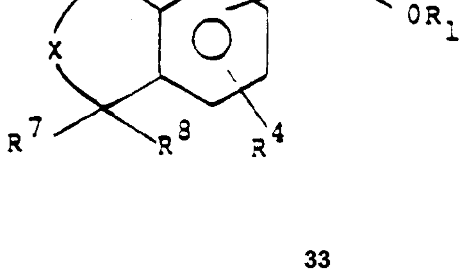

I

dans laquelle

| | |
|---|---|
| $R^1$, $R^2$ | représentent des groupes alkyle en C 1-C 4, alcényle en C 2-C 4, ou bien |
| $R^1 + R^2$ | représentent un groupe alkylène en C 2-C 4 éventuellement substitué par des groupes alkyle en C 1-C 4, alcényle en C 2-C 4, ou hydroxyalkyle en C 1-C 4 ou hydroxyalcényle en C 2-C 4, |
| $R^3$ | représente H, $CH_3$, |
| $R^4$ | représente H, un groupe alkyle en C 1-C 4, |
| $R^5$, $R^6$, $R^7$, $R^8$ | représentent H, $CH_3$, $C_2H_5$, $CH_2CH_2CH_3$, $CH(CH_3)_2$, |
| X | représente un groupe méthylène, éthylidène, propylidène, éthylène, propylène, 2,3-butylidène, |

le nombre total des atomes de carbone de $R^1$, $R^2$ est inférieur ou égal à 6,
le nombre total des atomes de carbone de $R^1$, $R^2$, $R^3$, $R^4$ est inférieur ou égal à 11,
le nombre total des atomes de carbone de $R^5$, $R^6$, $R^7$, $R^8$ est inférieur ou égal à 6,
à l'exclusion des composés I pour lesquels

| | |
|---|---|
| $R^3$ | représente H, |
| $R^4$ | représente H ou $CH_3$, |
| $R^5$, $R^6$, $R^7$, $R^8$ | représentent $CH_3$, |
| X | représente un groupe éthylène, et |
| $R^1$, $R^2$ | représentent des groupes alkyle en C 1-C 4. |

5. Composition de parfum, caractérisée en ce qu'elle contient du 2,4-diméthyl-2-(1,1,4,4-tétraméthyl-tétraline-6-yl)-1,3-dioxolanne.

6. Composition de parfum, caractérisée en ce qu'elle contient de la 7-(formyl-diéthyl-acétal)-1,1,2,4,4-pentaméthyl-tétraline.

7. Utilisation des composés de formule

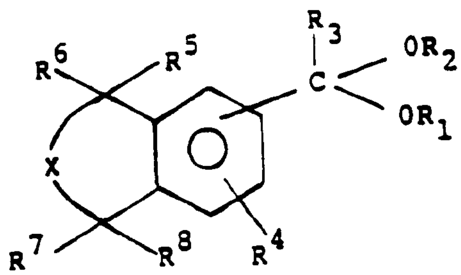

I

dans laquelle

| | |
|---|---|
| $R^1$, $R^2$ | représentent des groupes alkyle en C 1-C 4, alcényle en C 2-C 4, ou bien |
| $R^1 + R^2$ | représentent un groupe alkylène en C 2-C 4 éventuellement substitué par des groupes alkyle en C 1-C 4, alcényle en C 2-C 4, ou hydroxyalkyle en C 1-C 4 ou hydroxyalcényle en C 2-C 4, |
| $R^3$ | représente H, $CH_3$, |
| $R^4$ | représente un groupe alkyle en C 1-C 4, |
| $R^5$, $R^6$, $R^7$, $R^8$ | représentent H, $CH_3$, $C_2H_5$, $CH_2CH_2CH_3$, $CH(CH_3)_2$, |
| X | représente un groupe méthylène, éthylidène, propylidène, éthylène, propylène, 2,3-butylidène, |

le nombre total des atomes de carbone de $R^1$, $R^2$ est inférieur ou égal à 6,
le nombre total des atomes de carbone de $R^1$, $R^2$, $R^3$, $R^4$ est inférieur ou égal à 11,
le nombre total des atomes de carbone de $R^5$, $R^6$, $R^7$, $R^8$ est inférieur ou égal à 6,
à l'exclusion des composés I pour lesquels

| | |
|---|---|
| $R^3$ | représente H, |
| $R^4$ | représente H ou $CH_3$, |
| $R^5$, $R^6$, $R^7$, $R^8$ | représentent $CH_3$, |

X représente un groupe éthylène, et
$R^1$, $R^2$ représentent des groupes alkyle en C 1-C 4,
en tant que matières odorantes.

**8.** Utilisation du 2,4-diméthyl-2-(1,1,4,4-tétraméthyl-tétraline-6-yl)-1,3-dioxolanne en tant que matière odorante.

**9.** Utilisation de la 7-(formyl-diéthyl-acétal)-1,1,2,4,4-pentaméthyl-tétraline en tant que matière odorante.